# EUROPÄISCHE PATENTANMELDUNG

(11) **EP 4 342 300 A1**
(43) Veröffentlichungstag der Anmeldung: **27.03.2024**
(21) Anmeldenummer: 23189773.7
(22) Anmeldetag: 04.08.2023
(51) Int. Cl.: A23C 11/10, A23L 2/38, A23L 2/66, A23L 2/84, A23L 7/104, A23L 11/65, A23L 29/00, A23L 29/30, C12C 5/00, C12M 1/40, C12M 1/12

(54) **VERFAHREN ZUM HERSTELLEN ZUMINDEST EINES PFLANZENBASIERTEN GETRÄNKES UND VORRICHTUNG ZUM HERSTELLEN ZUMINDEST EINES PFLANZENBASIERTEN GETRÄNKES**

(30) Priorität: 21.09.2022 DE 102022124200
(71) Anmelder: frischli Milchwerke GmbH, 31547 Rehburg-Loccum (DE); Asepto GmbH Aseptische Verfahrenstechnik und Automation, 86473 Ziemetshausen (DE)
(72) Erfinder: Niemeyer, Sina Marie, 30163 Hannover (DE); Hesse, Jan, 31535 Neustadt (DE); Kaluza, Henrike, 31547 Rehburg (DE); Struckmann, Karl-Heinz, 31535 Neustadt (DE); Winkelmann, Timo, 31515 Wunstorf (DE); Eberhard, Martin, 89335 Ichenhausen - Deubach (DE); Westing, Christian, 86424 Dinkelscherben (DE)
(74) Vertreter: Jabbusch, Matthias

(57) **Zusammenfassung**

Bei einem Verfahren zum Herstellen zumindest eines pflanzenbasierten, insbesondere getreidebasierten Getränkes, bei dem wenigstens ein pflanzlicher Rohstoff mit Wasser vermengt und temperiert wird und der Rohstoff zumindest einer enzymatischen Behandlung unterworfen wird, ist vorgesehen, dass die enzymatische Behandlung entlang zumindest einer Fließstrecke für den Rohstoff durchgeführt wird.

Eine Vorrichtung zum Herstellen zumindest eines pflanzenbasierten, insbesondere getreidebasierten Getränkes, insbesondere zum Durchführen des vorgenannten Verfahrens, bei dem wenigstens ein pflanzlicher Rohstoff mit Wasser vermengt und temperiert wird und der Rohstoff zumindest einer enzymatischen Behandlung unterworfen wird, ist dadurch gekennzeichnet, dass sie für die enzymatische Behandlung zumindest eine Rohrstrecke aufweist.

## Beschreibung

Die Erfindung betrifft ein Verfahren zum Herstellen zumindest eines pflanzenbasierten, insbesondere getreidebasierten Getränkes, bei dem wenigstens ein pflanzlicher Rohstoff mit Wasser vermengt und temperiert wird und der Rohstoff zumindest einer enzymatischen Behandlung unterworfen wird. Die Erfindung betrifft des Weiteren eine Vorrichtung zum Herstellen zumindest eines pflanzenbasierten, insbesondere getreidebasierten Getränkes, insbesondere zum Durchführen des vorgenannten Verfahrens.

Getränke auf Kuhmilchbasis sind beispielsweise Trinkmilch, Kakao oder auch Fruchtmilcherzeugnisse. Für Laktoseintolerante, für vegan lebende Personen sind in den letzten Jahren pflanzenbasierte, insbesondere getreidebasierte Getränke entwickelt worden. Insbesondere auf Haferbasis sind beispielsweise milchartige Getränke entwickelt worden, reine Hafergetränke oder auch Hafermischgetränke. Auch andere pflanzliche Rohstoffe können zum Herstellen für vegane Personen geeigneter Getränke verwendet werden.

Im Stand der Technik ist in derartigen Verfahren schon vorgeschlagen worden, dass ein entsprechender pflanzlicher Rohstoff mit Wasser vermengt und temperiert, insbesondere erwärmt, wird. Anschließend wird der Rohstoff im Wasser zumindest einer enzymatischen Behandlung unterworfen. Dem Vermengen des Rohstoffs mit dem Wasser geht regelmäßig eine Zerkleinerung des Rohstoffs, beispielweise durch Mahlen, voraus. Die enzymatische Behandlung erfolgt ein- und mehrstufig, dabei oder anschließend kann ein Trenn- und Erhitzungsprozess folgen.

Diese Verfahrensschritte werden im Stand der Technik in einem sogenannten Batch-Verfahren durchgeführt. Das bedeutet, dass immer eine Charge eines herzustellenden Getränkes angesetzt wird, mit der die einzelnen Verfahrensschritte durchgeführt werden. Nach Abschluss eines Herstellungsverfahrens wird das hergestellte Getränk entnommen und es wird mit dem pflanzlichen Rohstoff und Wasser ein neuer Verfahrensablauf gestartet.

Der Erfindung liegt die Aufgabe zugrunde, ein Verfahren zum Herstellen eines pflanzenbasierten Getränkes der eingangs genannten Gattung aufzuzeigen, das den Batch-Betrieb vermeidet und unter Einsatz geringerer Energiemengen durchführbar ist. Weiterhin soll eine Vorrichtung zum Herstellen eines pflanzenbasierten Getränkes aufgezeigt werden, mit der insbesondere dieses Verfahren durchführbar ist.

Hinsichtlich des Verfahrens ist diese Aufgabe erfindungsgemäß dadurch gelöst, dass die enzymatische Behandlung entlang zumindest einer Fließstrecke für den Rohstoff durchgeführt wird.

Bei dem erfindungsgemäßen Verfahren wird der pflanzliche Rohstoff vermengt mit Wasser nicht in einen Behälter für einen Batch-Betrieb gegeben. Vielmehr ist zumindest eine Fließstrecke für den Rohstoff vorgesehen, wobei entlang dieser Fließstrecke die enzymatische Behandlung erfolgt. Das erfindungsgemäße Vorsehen zumindest einer Fließstrecke während der enzymatischen Behandlung ermöglicht eine Abkehr vom Batch-Betrieb und die Einrichtung eines kontinuierlichen Herstellungsbetriebes. Durch eine fortlaufende Beschickung der Fließstrecke mit Rohstoff, vermengt mit Wasser, kann ein kontinuierlicher Betrieb erreicht werden, mit dem die hergestellten Getränkemengen im Vergleich zum Batchverfahren erhöht werden können und eine homogenere Produktqualität haben.

Nach einer ersten Weiterbildung des Verfahrens ist vorgesehen, dass die enzymatische Behandlung entlang zumindest einer Fließstrecke mit wenigstens einem Temperaturhalteabschnitt durchgeführt wird. In der Fließstrecke ist mindestens ein Temperaturhalteabschnitt vorgesehen, in diesem Abschnitt kann ein hinzugefügtes Enzym auf das Gemenge von Rohstoff und Wasser einwirken.

Eine nächste Weiterbildung des Verfahrens sieht vor, dass dem Rohstoff Enzyme außerhalb eines Temperaturhalteabschnittes zugegeben werden. Nach dieser Weiterbildung können Enzyme dem Rohstoff entlang der Fließstrecke beispielsweise in einem Rohrabschnitt zugegeben werden, im Temperaturhalteabschnitt selbst erfolgt dann keine Zugabe mehr. In diesem Abschnitt können die Enzyme auf den Rohstoff einwirken, dazu wird in dem Temperaturhalteabschnitt eine dem Rohstoff und den Enzymen angepasste Temperatur gehalten.

In der Fließstrecke für den Rohstoff können mehrere Temperaturhalteabschnitte vorgesehen sein. Das Verfahren kann dann so ausgestaltet werden, dass ein bestimmtes Enzym erst dann dem Rohstoff zugegeben wird, wenn der Rohstoff bereits einen ersten Temperaturhalteabschnitt passiert hat. In diesem ersten Temperaturhalteabschnitt wirkt gegebenenfalls ein erstes hinzugegebenes Enzym auf den Rohstoff ein, ein zweites Enzym kann dann dem Rohstoff zwischen zwei Temperaturhalteabschnitten zugegeben werden.

Der für das Verfahren zum Herstellen des pflanzenbasierten Getränkes eingesetzte Rohstoff wird mit Wasser vorzugsweise in einem Temperaturbereich von 4 °C bis 95 °C vermengt. In diesem weiten Temperaturbereich ist eine Zugabe des Rohstoffs zu Wasser bis zu einer Konzentration von 30 % im Wasser möglich, eine Konzentration von 15 % bis 25 % ist bevorzugt.

Pflanzliche Rohstoffe sind insbesondere Getreide, wie Hafer, und/oder Pflanzenmehle, insbesondere von Hülsenfrüchten.

Auch bei dem erfindungsgemäßen Verfahren kann der pflanzliche Rohstoff vor dem Vermengen mit Wasser zerkleinert werden. Die Vermengung von Rohstoff und Wasser hat vorzugsweise eine Partikelgröße von etwa 50 µm bis 1.200 µm.

Der Rohstoff wird nach einer nächsten Weiterbildung der Erfindung verkleistert. Die Verkleisterung erfolgt vorzugsweise in einem Temperaturbereich von 60 °C bis 120 °C, vorzugsweise in dem Temperaturbereich 80 °C bis 95 °C.

Während der enzymatischen Behandlung entlang zumindest einer Fließstrecke kann nach einer Weiterbildung der Erfindung dem Rohstoff in seiner Vermengung mit Wasser, ein Enzym Alpha-Amylase zugegeben werden. Dieses Enzym setzt die Viskosität herab, die Vermengung bleibt dadurch über die Fließstrecke bringbar. Die eintretende Verflüssigung durch die Alpha-Amylase wird vorzugsweise in einem Temperaturbereich von 60 °C bis 90 °C durchgeführt. Diese Verflüssigung wird nach dem Verfahren vorzugsweise innerhalb eines zum Beispiel ersten Temperaturhalteabschnittes durchgeführt. Eine erste Enzymzugabe in das Wasser kann dabei schon vor dem Vermengen mit dem Rohstoff erfolgen. Die Zugabe des Enzyms kann auch vor dem Vermengen in das Wasser erfolgen (beides geschieht vor der sogenannten enzymatischen Behandlung).

Nach einer nächsten Weiterbildung der Erfindung werden dem Rohstoff Zucker bildende Enzyme zugegeben. Dies ist eine weitere, zweite enzymatische Behandlung, auch für diese weitere enzymatische Behandlung kann wieder ein Temperaturhalteabschnitt in der Fließstrecke vorgesehen sein. Die Zuckerbildenden Enzyme werden dem Rohstoff dann zwischen zwei Temperaturhalteabschnitten zugegeben. Dies erfolgt vorzugsweise in einem Temperaturbereich von 40 °C bis 65 °C.

Die Enzymierungszeiten der Alpha-Amylase und/oder der Zucker bildenden Enzyme kann auf 20 min bis 90 min eingestellt werden.

Für das erfindungsgemäße Verfahren kann schließlich noch vorgesehen sein, dass in der enzymatischen Behandlung weitere Enzyme zum Eiweißaufschluss und/oder zur Glutenreduktion und/oder zur Anpassung der Konsistenz des hergestellten Getränks und/oder zur Anreicherung bestimmter Nährstoffe zugegeben werden.

Enzymierungszeiten, Temperaturniveaus und auch Dosiermengen während der enzymatischen Behandlung sind der gewünschten Endproduktqualität anzupassen. Übliche Dosiermengen für Alpha-Amylasen sind 2.000 ppm bis 4.000 ppm und für Glucosidasen 1.000 ppm bis 2.000 ppm. Diese Angaben beziehen sich je nach Hersteller auf den Trockenstoff- oder den Stärkeanteil der Rohstoff-Wassermischung.

Die vorrichtungsseitige Lösung der Aufgabe, für die selbständiger Schutz beansprucht wird, ist dadurch gekennzeichnet, dass sie für die enzymatische Behandlung zumindest eine Rohrstrecke aufweist.

Nach dem erfindungsgemäßen Verfahren ist eine Fließstrecke für den Rohstoff vorgesehen, entlang der die enzymatische Behandlung erfolgt. Die Vorrichtung stellt für diese Fließstrecke zumindest eine Rohrstrecke zur Verfügung, dabei können die Rohre voneinander verschiedene Querschnitte und auch Ausbildungen haben. Der Rohstoff wird durch die Rohrstrecke geführt und der enzymatischen Behandlung unterworfen.

Eine erste Weiterbildung der erfindungsgemäßen Vorrichtung sieht zumindest eine Rohrstrecke für Wasser vor, wobei in diese Rohrstrecke zumindest eine Zugabe eines Rohstoffs einmündet. Während des Verfahrens wird ein Gemenge aus Rohstoff und Wasser hergestellt. Die Vorrichtung sieht dafür zunächst eine Rohrstecke für Wasser vor, in die der Rohstoff hinzugegeben wird.

Vorzugsweise geht die Rohrstrecke für Wasser in die Rohrstrecke für die enzymatische Behandlung über, es handelt sich also um ein und dieselbe Rohrstrecke. Weiter vorzugsweise mündet in Fließrichtung des Wassers vor und/oder nach der Zugabe des Rohstoffs zumindest eine Aufgabe eines Enzyms in die Rohrstrecke ein. Die enzymatische Behandlung kann bei der erfindungsgemäßen Vorrichtung durch ein Zugeben eines Enzyms vor oder nach dem Einführen des Rohstoffs in das Wasser erfolgen. Entsprechende Aufgaben mit Tanks, Rohrleitungen und Ventilen für das Enzym sind der Rohrstrecke zugeordnet.

Nach einer nächsten Weiterbildung der Erfindung ist vorgesehen, dass die Vorrichtung in der Rohrstrecke zumindest einen Temperaturhalteabschnitt aufweist. Durch einen derartigen Temperaturhalteabschnitt wird der Rohstoff geführt, Enzyme wirken auf ihn ein. Dabei kann vorgesehen sein, dass ein zweiter Temperaturhalteabschnitt in die Rohrstrecke eingesetzt ist und dass zwischen beiden Temperaturhalteabschnitten zumindest eine Aufgabe eines Enzyms in die Rohrstrecke einmündet. Ein Enzym kann dem Rohstoff in der Rohrstrecke vor dem ersten Temperaturhalteabschnitt zugeführt werden, ein zweites Enzym dann in dem Abschnitt der Rohrstrecke zwischen den beiden Temperaturhalteabschnitten zugegeben werden.

Vorzugsweise weist jeder Temperaturhalteabschnitt eine Vielzahl von Rohren auf. Die Rohrstrecke kann beispielsweise aus einem Rohr bestehen, der Temperaturhalteabschnitt dagegen aus vielen Rohren. Diese Rohre sind vorzugsweise nacheinander in die Rohrstrecke eingesetzt, so dass der Rohstoff nicht auf viele Rohre aufgeteilt wird und nicht parallel durch diese fließt, sondern dass die Rohre in Reihe ein nach dem anderen von dem Rohstoff durchflossen werden. Der Durchmesser der Rohre kann dabei 18 mm bis 160 mm betragen, angepasst an den zu verarbeitenden Rohstoff. Als Material für die Rohre kann Edelstahl verwendet werden. Weiter kann vorgesehen sein, dass in die Rohre Ventile eingesetzt sind oder dass ein Koppelpaneel zur Verbindung von Rohrenden vorgesehen ist. So können einzelne Abschnitte der Rohrstrecke weg oder dazu gekoppelt werden. Dadurch lassen sich voneinander verschiedene Enzymierungszeiten erreichen, eine Reaktion auf Enzym- oder Rohstoffänderungen ist möglich.

Nach einer nächsten Weiterbildung der Erfindung ist vorgesehen, dass die Rohre ringförmig gebogen sind und in Windungen übereinander angeordnet sind. Der Abstand einander benachbarter Rohre kann dabei geringgehalten werden, so dass sich energetische Effekte zwischen den Rohren ergeben. Dies auch dann, wenn von außen Wärme zugeführt wird.

Es können dabei Rohre mit voneinander verschiedenen ringförmigen Biegungen verwendet werden und in Windungen übereinander und ineinander angeordnet werden. Es gelingt so ein nahes Packen der Windungen, gleichwohl wird eine lange Rohrstrecke insbesondere für die enzymatische Behandlung des Rohstoffs bereitgestellt.

Im Inneren der Windungen können Steigleitungen angeordnet sein, mit denen das Gemenge aus Rohstoff und Wasser weiter zur nächsten Windung führen. Die Windungen selbst werden von oben nach unten mit dem Rohstoff durchflossen, um Absetzungen des Rohstoffs zu vermeiden. Die Steigleitungen können dabei geringere Durchmesser als die Rohre der Windungen aufweisen. Dadurch wird der Rohstoff beim Durchfließen der Steigleitungen beschleunigt und durchmischt.

Alternativ kann ein Temperaturhalteabschnitt auch Rohre aufweisen, die parallel zueinander ausgerichtet sind und im Paket über- und nebeneinander mit Umlenkabschnitten um 180° an den Rohrenden angeordnet sind. Ein derartiger Temperaturhalteabschnitt kann beispielsweise insgesamt quaderförmig ausgebildet sein, er kann aus einem Rohrpaket bestehen. Ein nacheinander Durchfließen der Rohre wird durch die Umlenkabschnitte um 180° sichergestellt. Auch bei diesem Temperaturhalteabschnitt können die Rohre eng zueinander angeordnet sein, ein kompaktes Eintragen von Wärme sowie eine Verbreitung von Wärme innerhalb des Paketes ist vorteilhaft gewährleistet.

Der Durchmesser der Umlenkabschnitte ist etwa gleich dem 2- bis 5fachen der Rohrdurchmesser.

Zur weiteren Ausbildung der erfindungsgemäßen Vorrichtung kann noch vorgesehen sein, dass die Rohrstrecke eine Wärmeisolierung aufweist. Das Verfahren zum Herstellen des Getränkes verlangt zum Teil höhere Temperaturen, das Vorhalten dieser wird gegenüber einer Umgebung isoliert. Ausführungsbeispiele der Erfindung sind in der Zeichnung dargestellt. Es zeigen:
- Figur 1:: ein schematisches Fließbild einer erfindungsgemäßen Vorrichtung zum Herstellen zumindest eines pflanzenbasierten Getränkes;
- Figur 2:: eine perspektivische Ansicht eines Bauteils der Vorrichtung gemäß Figur 1;
- Figur 3 und 3a:: Schnittansichten des Bauteils gemäß Figur 2;
- Figur 4:: eine perspektivische Ansicht einer alternativen Ausführungsform eines Bauteils der Vorrichtung gemäß Figur 1 und
- Figur 5:: eine Seitenansicht des Bauteils gemäß Figur 4.

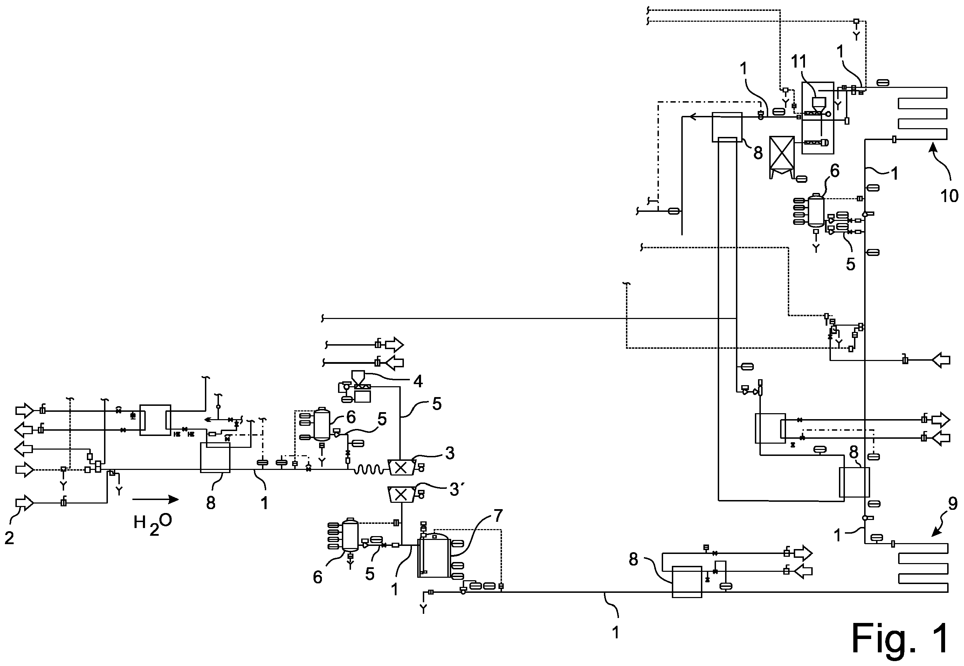

Die Vorrichtung in Figur 1 weist eine Rohrstrecke 1 auf. In die Rohrstrecke 1 wird Wasser eingegeben, wie mit dem Pfeil 2 verdeutlicht. In einem Gerät zur gegebenenfalls Zerkleinerung und Vermischung 3 erfolgt ein Aufgeben eines pflanzlichen Rohstoffs in die Rohrstrecke 1. Dazu wird der pflanzliche Rohstoff aus einem Vorratsbehälter 4 in eine Teilleitung 5 geführt, die Teilleitung 5 verläuft zu dem Gerät 3. Mit einem weiteren Gerät 3' kann noch eine weitere Vermischung des Rohstoffs erfolgen. Im Bereich dieser Aufgabe des Rohstoffs in die Rohrstrecke 1 sind Behälter 6 für ein erstes Enzym angeordnet. Die Behälter 6 haben wieder Teilleitungen 5, die in die Rohrstrecke 1 einmünden.

Die Rohrstrecke 1 wird anschließend durch ein Vorlaufgefäß 7 geführt und mittels eines Wärmetauschers 8 erwärmt. Ein erster Wärmetauscher 8 ist schon in der Rohrstrecke 1 für das Wasser vor Aufgabe des Rohstoffs angeordnet. Im Bereich der Geräte 3, 3' kann das Wasser bis auf 95 °C erwärmt sein. Bei dem Wärmetauscher 8 in Fließrichtung hinter dem Vorlaufgefäß 7 wird die Temperatur in der Rohrstrecke 1 auf etwa zum Beispiel 85 °C eingestellt.

Die Rohrstrecke 1 mündet nach dem zweiten Wärmetauscher 8 in einen ersten Temperaturhalteabschnitt 9 ein. Nach Austritt aus diesem Temperaturhalteabschnitt 9 passiert die Rohrstrecke 1 einen weiteren Wärmetauscher 8 und einen weiteren Behälter 6 für ein anderes Enzym. Anschließend wird die Rohrstrecke 1 in einen zweiten Temperaturhalteabschnitt 10 geführt.

Das aus dem zweiten Temperaturhalteabschnitt 10 über die Rohrstrecke 1 austretende insoweit hergestellte pflanzenbasierte Getränk wird noch über einen Dekanter 11 zum Ausscheiden grober Partikel und über die Rohrstrecke 1 über einen weiteren Wärmetauscher 8 zu einer weiteren Verarbeitung und Abfüllung geführt.

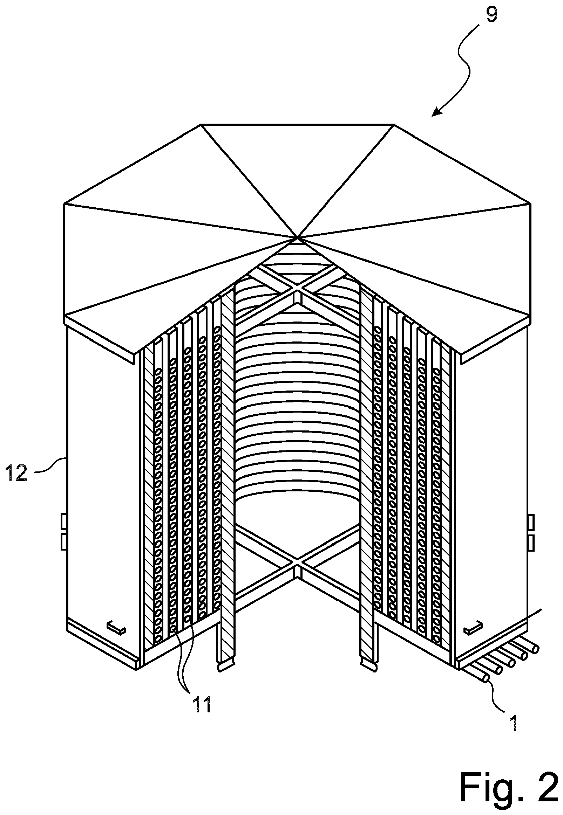

Figur 2 zeigt einen Temperaturhalteabschnitt, beispielsweise den Temperaturhalteabschnitt 9. Dieser Temperaturhalteabschnitt 9 weist eine Vielzahl von Rohren 11 auf, die eng aufeinandergelegt sind und in konzentrischen Ringen angeordnet sind. Die Rohrstrecke 1 mündet in diesen Temperaturhalteabschnitt 9 mit den vielen Rohren 11 ein und wird in Reihe durch diese Rohre 11 geführt. Der Temperaturhalteabschnitt 9 weist dabei eine abnehmbare Verkleidung 12 auf.

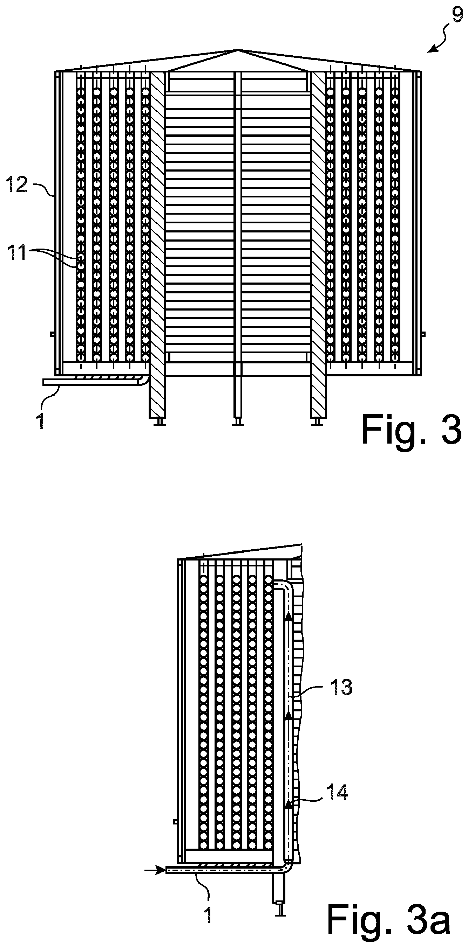

Auch Figur 3 zeigt die aufeinanderliegenden Rohre 11. Die Rohrstrecke 1 wird von unten in den Temperaturhalteabschnitt 9 eingeführt, danach führen Steigleitungen im Inneren nach oben. Eine Steigleitung 13 ist in Figur 3a gezeigt. Der zugeführte Rohstoff wird entlang Pfeil 14 nach oben geführt und durchfließt dann nacheinander die Rohre 11. Trotz kompakter Bauform wird eine Rohrstrecke 1 von über 1.000 m zur Verfügung gestellt.

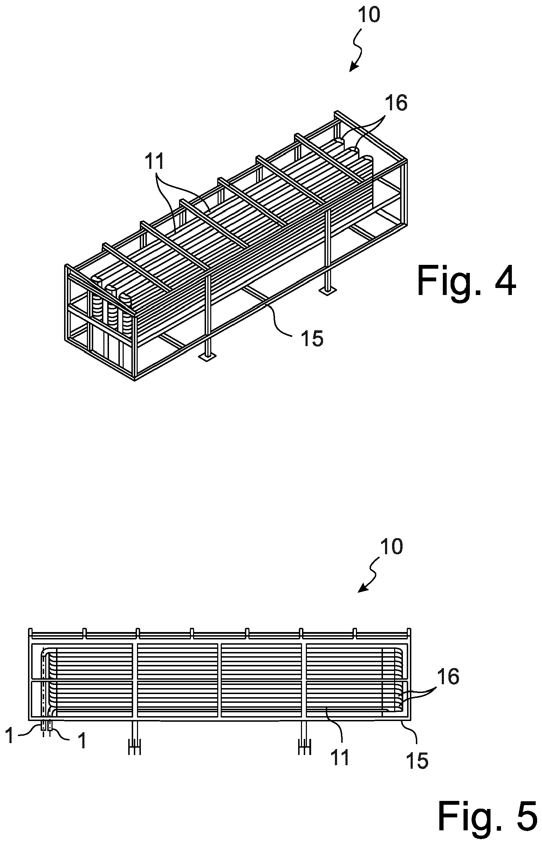

Eine alternative Ausbildung für einen Temperaturhalteabschnitt 9,10, beispielsweise für den Temperaturhalteabschnitt 10 zeigt Figur 4. Hier sind die Rohre 11 parallel zueinander ausgerichtet und im Paket über und nebeneinander angeordnet. Für die Rohre 11 ist ein Haltegestell 15 vorgesehen. An den Enden der Rohre 11 sind Umlenkabschnitte 16 um 180° angeordnet, so dass auch hier die Rohre 11 von dem Rohstoff nacheinander durchflossen werden.

Figur 5 zeigt Ein- und Austritt der Rohrstrecke 1 in den Temperaturhalteabschnitt 10.

## Patentansprüche

1. Verfahren zum Herstellen zumindest eines pflanzenbasierten, insbesondere getreidebasierten Getränkes, bei dem wenigstens ein pflanzlicher Rohstoff mit Wasser vermengt und temperiert wird und der Rohstoff zumindest einer enzymatischen Behandlung unterworfen wird,
**dadurch gekennzeichnet,**
**dass** die enzymatische Behandlung entlang zumindest einer Fließstrecke für den Rohstoff durchgeführt wird.

2. Verfahren nach Anspruch 1, **dadurch gekennzeichnet, dass** die enzymatische Behandlung entlang zumindest einer Fließstrecke mit wenigstens einem Temperaturhalteabschnitt (9,10) durchgeführt wird.

3. Verfahren nach Anspruch 2, **dadurch gekennzeichnet, dass** dem Rohstoff Enzyme außerhalb eines Temperaturhalteabschnittes (9,10) zugegeben werden.

4. Verfahren nach Anspruch 2 und 3, **dadurch gekennzeichnet, dass** dem Rohstoff Enzyme zwischen zwei Temperaturhalteabschnitten (9,10) zugegeben werden.

5. Verfahren nach einem der vorhergehenden Ansprüche, **dadurch gekennzeichnet, dass** der pflanzliche Rohstoff mit Wasser in einem Temperaturbereich von 4°C bis 95°C vermengt wird.

6. Verfahren nach einem der vorhergehenden Ansprüche, **dadurch gekennzeichnet, dass** der pflanzliche Rohstoff dem Wasser bis zu einer Konzentration von 30% im Wasser zugegeben wird.

7. Verfahren nach einem der vorhergehenden Ansprüche, **dadurch gekennzeichnet, dass** als pflanzliche Rohstoffe Getreide, wie Hafer, und/oder Pflanzenmehle insbesondere von Hülsenfrüchten eingesetzt werden.

8. Verfahren nach einem der vorhergehenden Ansprüche, **dadurch gekennzeichnet, dass** die Vermengung von Rohstoff und Wasser auf eine Partikelgröße von etwa 50 µm bis 1200 µm eingestellt wird.

9. Verfahren nach einem der vorhergehenden Ansprüche, **dadurch gekennzeichnet, dass** der Rohstoff verkleistert wird.

10. Verfahren nach einem der Ansprüche 3 bis 9, **dadurch gekennzeichnet, dass** dem Rohstoff ein Enzym alpha-Amylase zugegeben wird.

11. Verfahren nach Anspruch 10, **dadurch gekennzeichnet, dass** das Enzym alpha-Amylase in einem Temperaturbereich von 60°C bis 90°C zugegeben wird.

12. Verfahren nach Anspruch 11, **dadurch gekennzeichnet, dass** die Verkleisterung in einem Temperaturbereich von 60°C bis 120°C, vorzugsweise von 80°C bis 95°C durchgeführt wird.

13. Verfahren nach einem der Ansprüche 3 bis 12, **dadurch gekennzeichnet, dass** dem Rohstoff Zucker bildende Enzyme zugegeben werden.

14. Verfahren nach Anspruch 12, **dadurch gekennzeichnet, dass** die Zucker bildenden Enzyme in einem Temperaturbereich von 40°C bis 65°C zugegeben werden.

15. Verfahren nach einem der Ansprüche 9 bis 14, **dadurch gekennzeichnet, dass** die Enzymierungszeit der alpha-Amylase und/oder die Zucker bildenden Enzyme auf 20 min bis 90 min eingestellt wird.

16. Verfahren nach einem der Ansprüche 3 bis 15, **dadurch gekennzeichnet, dass** in der enzymatischen Behandlung weitere Enzyme zum Eiweißaufschluss und/oder zur Glutenreduktion und/oder zur Anpassung der Konsistenz des hergestellten Getränks zugegeben werden.

17. Vorrichtung zum Herstellen zumindest eines pflanzenbasierten, insbesondere getreidebasierten Getränkes, insbesondere zum Durchführen des Verfahrens nach einem der Ansprüche 1 bis 16, bei dem wenigstens ein pflanzlicher Rohstoff mit Wasser vermengt und temperiert wird und der Rohstoff zumindest einer enzymatischen Behandlung unterworfen wird,
**dadurch gekennzeichnet,**
**dass** sie für die enzymatische Behandlung zumindest eine Rohrstrecke (1) aufweist.

18. Vorrichtung nach Anspruch 17, **dadurch gekennzeichnet, dass** sie zumindest eine Rohrstrecke (1) für Wasser hat und dass in diese Rohrstrecke (1) zumindest eine Zugabe eines Rohstoffs einmündet.

19. Vorrichtung nach Anspruch 17, **dadurch gekennzeichnet, dass** die Rohrstrecke (1) für Wasser die Rohrstrecke (1) für die enzymatische Behandlung ist und dass in Fließrichtung des Wassers vor und/oder nach der Zugabe des Rohstoffs zumindest eine Aufgabe eines Enzyms in die Rohrstrecke (1) einmündet.

20. Vorrichtung nach einem der Ansprüche 17 bis 19, **dadurch gekennzeichnet, dass** die Rohrstrecke (1) zumindest einen Temperaturhalteabschnitt (9,10) aufweist.

21. Vorrichtung nach Anspruch 20, **dadurch gekennzeichnet, dass** ein zweiter Temperaturhalteabschnitt (9,10) in die Rohrstrecke (1) eingesetzt ist und dass zwischen beiden Temperaturhalteabschnitten (9,10) zumindest eine Aufgabe eines Enzyms in die Rohrstrecke (1) einmündet.

22. Vorrichtung nach Anspruch 20 oder 21, **dadurch gekennzeichnet, dass** jeder Temperaturhalteabschnitt (9,10) eine Vielzahl von Rohren (11) aufweist.

23. Vorrichtung nach Anspruch 22, **dadurch gekennzeichnet, dass** der Durchmesser der Rohre (11) 18 mm bis 160 mm beträgt.

24. Vorrichtung nach Anspruch 22 oder 23, **dadurch gekennzeichnet, dass** für die Rohre (11) Edelstahl als Materialien eingesetzt werden.

25. Vorrichtung nach einem der Ansprüche 20 bis 24, **dadurch gekennzeichnet, dass** in die Rohre (11) Ventile eingesetzt sind.

26. Vorrichtung nach einem der Ansprüche 22 bis 25, **dadurch gekennzeichnet, dass** den Rohren (11) zumindest ein Koppelpaneel zur Verbindung von Rohrenden zugeordnet ist.

27. Vorrichtung nach einem der Ansprüche 22 bis 26, **dadurch gekennzeichnet, dass** die Rohre (11) ringförmig gebogen sind und in Windungen übereinander angeordnet sind.

28. Vorrichtung nach Anspruch 27, **dadurch gekennzeichnet, dass** die Rohre (11) mit voneinander verschiedenen ringförmigen Biegungen verwendet werden und in Windungen übereinander und ineinander angeordnet werden.

29. Vorrichtung nach Anspruch 27 oder 28, **dadurch gekennzeichnet, dass** im Inneren der Windungen Steigleitungen zur nächsthöheren Windung angeordnet sind.

30. Vorrichtung nach Anspruch 29, **dadurch gekennzeichnet, dass** die Steigleitungen geringere Durchmesser als die Rohre (11) der Windungen aufweisen.

31. Vorrichtung nach einem der Ansprüche 22 bis 26, **dadurch gekennzeichnet, dass** die Rohre (11) parallel zueinander ausgerichtet sind und im Paket über- und nebeneinander mit Umlenkabschnitten (14) um 180° an den Rohrenden angeordnet sind.

32. Vorrichtung nach Anspruch 31, **dadurch gekennzeichnet, dass** der Durchmesser der Umlenkabschnitte (16) etwa gleich dem 2- bis 5fachen der Rohrdurchmesser ist.

33. Vorrichtung nach einem der Ansprüche 17 bis 32, **dadurch gekennzeichnet, dass** die Rohrstrecke (1) eine Wärmeisolierung aufweist.
